# EUROPEAN PATENT SPECIFICATION

(11) **EP 1 049 461 B1**
(45) Date of publication and mention of the grant of the patent: **09.01.2002**
(21) Application number: 99902686.7
(22) Date of filing: 28.01.1999
(51) Int. Cl.: A61K 31/21, A61F 6/04

(54) **USE OF TOPICAL PREPARATIONS OF GLYCERYL TRINITRATE AND LANOLIN FOR THE TREATMENT OF ERECTILE DYSFUNCTION**
VERWENDUNG VON TOPISCHE PRÄPARATIONEN MIT GLYCERYL TRINITRAT UND LANOLIN ZUR BEHANDLUNG VON EREKTILEN FUNKTIONSSTÖRUNGEN
UTILISATION DE PREPARATIONS TOPIQUES CONTENANT DE LA GLYCERYL TRINITRALE ET DE LA LANOLINE POUR TRAITER LES TROUBLES DE LA FONCTION ERECTILE

(30) Priority: 30.01.1998 GB 9802078
(43) Date of publication of application: 08.11.2000
(73) Proprietor: Futura Medical Limited, Petersfield, Hampshire GU31 4AD (GB)
(72) Inventor: Kemp, Colin Anthony, Havant, Hampshire PO9 1AX (GB)
(74) Representative: Harrison, Ivor Stanley
(86) International application number: GB9900288
(87) International publication number: WO9938506

(56) References cited:
- US-A- 4 450 175
- US-A- 4 822 617
- US-A- 4 829 991
- US-A- 5 292 512
- US-A- 5 470 563
- US-A- 5 565 466
- US-A- 5 698 589
- DATABASE WPI Week 9038 Derwent Publications Ltd., London, GB; AN 90-286014 XP002066221 & JP 02 200633 (AKAMATSU SHOJI KK) , 8 August 1990 & JP 02 200633 A (AKAMATSU SHOJI KK)
- REYNOLDS (ED.): "Martindale - The Extra Pharmacopoeia" 1996 , ROYAL PHARMACEUTICAL SOCIETY , LONDON XP002106988 see page 874-877 see page 874, middle column, paragraph 4 see page 876, left-hand column, paragraph 5
- HOUSE AND WAGLE: "In Vivo and In Vitro Correlation of the Role of the Ointment Composition in Percutaneous Absorption of Nitroglycerin" TODAY'S THERAPEUTIC TRENDS, vol. 2, no. 1, 1984, pages 7-15, XP002066218
- CHEMICAL ABSTRACTS, vol. 98, no. 10, 1982 Columbus, Ohio, US; abstract no. 78030, GUI ET AL: "Percutaneous Absorption Test of Several Nitroglycerin Ointments Through Human Skin" XP002066220 & GUI ET AL: YAOXUE TONGBAO, vol. 17, no. 11, 1982, pages 679-681,
- ZUGERMAN ET AL: "Allergic Contact Dermatitis Secondary to Nitroglycerin in Nitro-Bid Ointment" CONTACT DERMATITIS, vol. 5, no. 4, 1979, pages 270-271, XP002066217
- PATENT ABSTRACTS OF JAPAN vol. 004, no. 113 (C-021), 13 August 1980 & JP 55 072108 A (SANWA KAGAKU KENKYUSHO KK)
- DATABASE WPI Week 9610 Derwent Publications Ltd., London, GB; AN 96-096007 XP002066222 & RU 2 036 644 (LORAN O.B.), 9 June 1995 & RU 2 036 644 A (LORAN O.B.)
- OWEN ET AL: 'Topical Nitroglycerin: A Potential Treatment For Impotence' THE JOURNAL OF UROLOGY vol. 141, March 1989, pages 546 - 548
- MEYHOFF ET AL: 'Non-Invasive Management of Impotence with Transcutaneous Nitroglycerin' BRITISH JOURNAL OF UROLOGY vol. 69, 1992, pages 88 - 90
- S. BUDAVARI, ED.: 'Merck Index, entries 7155, 7326, 7327', 1996, MERCK & CO INC.
- VIDAL: 'EDITIONS DU VIDAL', 1996, PARIS see page 887, column LEFT

## Description

This invention relates to a preparation for topical application to the male sexual organ, especially for the treatment of erectile dysfunction.

Erectile dysfunction is a widespread problem with human males, albeit not commonly discussed in public. It is not regarded as a medical condition susceptible to treatment in the conventional sense being to a large extent caused by a mental condition or at least exacerbated by a loss of confidence following an initial experience caused perhaps by tiredness or an over-indulgence in food or alcohol. Nevertheless, the consequences are serious to the sufferer, resulting in psychological damage and in many cases frustrating the normal fertilisation processes so that artificial insemination procedures must be used if procreation is required. Procedures for overcoming this problem have been proposed which can involve the surgical implantation of stiffening devices as described in GB-A-1476804, US-A-5242391 and other patents. Chemotherapeutical approaches have been proposed which require the injection of vasodilator compositions immediately prior to intercourse. The injection takes place on or near the penile shaft. Neither procedure is attractive and both approaches can result in revulsion or distress on the part of the partners involved. US-A-5059603 and US-A-4801587 propose topical application of compositions containing vasodilators. US-A-5059603 proposes the use of a composition including a combination of a vasodilator and vasoconstrictor in a carrier intended to assist absorption of the active ingredients through the skin of the penis, the active ingredients being chosen so that the vasodilator acts more quickly than the vasoconstrictor whereby an erection of the penis, having been initiated by the action of the vasodilator, is sustained by the action of the vasoconstrictor. Vasodilators said to be appropriate for use in such compositions include nitroglycerin, papaverine, hydralazine, sodium nitroprusside and phenoxybenzamine, among others. Vasoconstrictors include caffeine, 3-(2-aminoethyl)indole derivatives and adrenaline. US-A-4801587 proposes a composition in the form of an ointment and including a vasolidator selected from papaverine, hydralazine, sodium nitroprusside, phenoxybenzamine and phentolamine mixed in a base with dimethylsulfoxide as a skin-absorption agent. Dimethylsulfonide is also proposed for the same purpose in US-A-5059603.
In Derwent Abstract No. 90-286014 (corresponding to JP-A-2200633) a composition for the topical treatment of impotence is disclosed which comprises nitroglycerin and/or isosorbide dinitrate. In addition, however, this composition contains papaverine. It is also known (from *Martindale: The Extra Pharmacopoeia,* 1996, pp 874 to 877 and 2157. The Pharmaceutical Press, London) that topical nitroglycerin, in various formulations, can be used to produce penile erection in some subjects. However, the incidence of side effects such as headache is high using these prior formulations and thus, as a safety precaution, the user is required to wear a condom to protect his partner from unwanted effects.

A formulation containing nitroglycerin, lanolin and petrolatum has been shown to be capable of inducing beneficial effects in hypertension and angina pectoris patients (House and Wagle, 1984. *Today's Therapeutic Treads*, Vol. 2, pp 7 to 15). Similarly, formulations of nitroglycerin with lanolin, petrolatum and other excipients have been shown to lead to absorption of nitroglycerin following topical application to skin (Chemical Abstract No. 98:78030; corresponding to Gui *et al*, 1982. *Yaoxue Tongbao*, Vol. 17, pp 678 to 681). Neither of these latter two documents, however, includes reference to the potential usefulness of the described formulations in impotence therapy.

US-A-4829991 describes a method and device for stimulating penile erection. A condom is coated on its interior surface with a commercially available nitroglycerine formulation. The application of the condom to the penis is said to stimulate and maintain an erection.

It has now been found that nitroglycerin (glyceryl trinitrate) can be used as a vasodilator in a composition for topical application to the penis in conjunction with carrier materials without either a vasoconstrictor or dimethyl sulfoxide, which is known to be a toxic material, and that such compositions are effective for the treatment of erectile dysfunction.

According to one aspect of the present invention, there is provided the use of a combination of compounds in the preparation of a composition for topical application to the penis for the treatment of erectile dysfunction in human males, the compounds comprising glyceryl trinitrate as the sole active ingredient and lanolin.

The application of the composition before sexual intercourse results in an erection of the penis which not only enables subsequent intercourse to take place but also provides for the subject male a mental state of renewed confidence in his ability as a sexual partner and may provide for the other partner a sense of relief from any self-doubt concerning her abilities to stimulate her partner to a state of sexual arousal.
The composition prepared according to the present invention may be used in conjunction with a condom. Accordingly, a further aspect of the invention provides, in combination, a composition for topical application to the penis for the treatment of erectile dysfunction in human males, the composition comprising glyceryl trinitrate as the sole active ingredient and lanolin, and a condom.

The glyceryl trinitrate acts as a vasodilator according to its established utility for example in the treatment of angina pectoris but it has not hitherto been recognised that in simple combination with lanolin there could be provided an effective treatment of erectile dysfunction in human males.

Glyceryl trinitrate is a material which is per se explosive. For use in the present invention, it is preferably adsorbed on a solid stabilizer in finely-divided particulate form, the particles being in the sub-micron size range to render them capable of passage through the skin. A preferred stabilizer compound is lactose on which the glyceryl trinitrate may be adsorbed at a concentration of from 5 to 20% by weight, for example 10% by weight.

The lanolin acts as a lubricant or moisturiser and also enhances skin absorption. It is known to be non-toxic and for the purpose of the present invention should be used as a medical grade such as "Medilan" (Trade Mark).

Preferably, compositions according to the invention also include a physical stabilizer such as a paraffin cream; optionally, the compositions contain other ingredients such as fragrances and/or colorants. The paraffin cream, for example provided as White Soft Paraffin B.P., promotes stability of the composition on the skin and also acts as a water-repellant barrier and lubricant.

The composition also includes water to adjust the overall viscosity or consistency and concentration of the active ingredient to the desired levels, bearing in mind that for ease of use the composition should be in the form of a physically-stable suspension of the active ingredient in a creamy emulsion which is sufficiently low in viscosity to be readily applied to and spread over the skin of the penis at ordinary body temperatures.

An example of a composition for use according to the present invention is given below:

| Ingredient | Weight % |
|---|---|
| Glyceryl trinitrate (10% in lactose) | 10 |
| Lanolin ("Medilan") | 44 |
| White Soft Paraffin B.P. | 21 |
| Demineralized Water | 25 |

In use, two grams of the above formulation, which has the form of a white cream, was applied to the glans penis and penile shaft and gently massaged into the skin. The effect was to produce an erection of the penis which was sustained for approximately 45 minutes. The effect in producing an erection way be enhanced by other stimuli of tactile, audio and/or visual nature.

The formulation may be used in conjunction with a condom, in which the formulation is applied to the penis and a condom applied in the usual manner after an erection has been attained. Condoms suitable for use with the formulation are preferably of the non-latex type, for example formed from polyurethane.

## Claims

1. Use of a combination of compounds in the preparation of a composition for topical application to the penis for the treatment of erectile dysfunction in human males, the compounds comprising glyceryl trinitrate as the sole active ingredient and lanolin.

2. The use as claimed in claim 1, in which the glyceryl trinitrate is absorbed on a solid stabilizer in finely-devided particulate form.

3. The use as claimed in claim 2, in which the stabilizer compound comprises lactose.

4. The use according to claim 3, in which the glyceryl trinitrate is absorbed on the lactose at a concentration of from 5 to 20% by weight.

5. The use according to any preceding claim, in which the composition also includes a physical stabilizer.

6. The use according to claim 5, in which the composition also includes water.

7. The use according to any preceding claim, in which the composition consists essentially of 1wt% glyceryl trinitrate (10wt% glyceryl trinitrate adsorbed on 10wt% lactose), 44wt% lanolin, 21wt% white soft paraffin B.P. and 25wt% demineralized water.

8. The use according to any preceding claim, wherein the topical application is in conjunction with the use of a condom.

9. In combination with a condom, a composition for topical application to the penis for the treatment of erectile dysfunction in human males, the composition comprising glyceryl trinitrate as the sole active ingredient and lanolin.

## Patentansprüche

1. Verwendung einer Kombination von Verbindungen bei der Bereitung einer Zusammensetzung zur lokalen Anwendung am Penis zur Behandlung von Erektionsstörungen bei Männern, wobei die Verbindungen Glycerintrinitrat als einzigen wirksamen Inhaltsstoff sowie Lanolin umfassen.

2. Verwendung nach Anspruch 1, wobei das Glycerintrinitrat an einem feststofflichen Stabilisator in feinverteilter Teilchenform absorbiert ist.

3. Verwendung nach Anspruch 2, wobei die Stabilisatorverbindung Lactose umfaßt.

4. Verwendung nach Anspruch 3, wobei das Glycerintrinitrat an der Lactose in einer Konzentration von 5 bis 20 Gewichtsprozent absorbiert ist.

5. Verwendung nach einem beliebigen vorangehenden Anspruch, wobei die Zusammensetzung ferner einen physikalischen Stabilisator umfaßt.

6. Verwendung nach Anspruch 5, wobei die Zusammensetzung ferner Wasser umfaßt.

7. Verwendung nach einem beliebigen vorangehenden Anspruch, wobei die Zusammensetzung im wesentlichen aus 1 Gewichtsprozent Glycerintrinitrat (10 Gewichtsprozent Glycerintrinitrat, adsorbiert an 10 Gewichtsprozent Lactose), 44 Gewichtsprozent Lanolin, 21 Gewichtsprozent weißen Weichparaffins B.P. und 25 Gewichtsprozent entmineralisierten Wassers besteht.

8. Verwendung nach einem beliebigen vorangehenden Anspruch, wobei die lokale Anwendung in Verbindung mit der Verwendung eines Kondoms erfolgt.

9. Zusammensetzung zur lokalen Anwendung am Penis zur Behandlung von Erektionsstörungen bei Männern in Verbindung mit einem Kondom, wobei die Zusammensetzung Glycerintrinitrat als einzigen wirksamen Inhaltsstoff sowie Lanolin umfaßt.

## Revendications

1. Utilisation d'une combinaison de composés dans la préparation d'une composition pour application topique au pénis pour le traitement des troubles de la fonction érectile chez les mâles humains, **caractérisée en ce que** les composés comprennent du trinitrate de glycéryle en tant qu'unique ingrédient actif et de la lanoline.

2. Utilisation selon la revendication 1, **caractérisée en ce que** le trinitrate de glycéryle est absorbé sur un stabilisant solide sous la forme de particules finement divisées.

3. Utilisation selon la revendication 2, **caractérisée en ce que** le stabilisant comprend du lactose.

4. Utilisation selon la revendication 3, **caractérisée en ce que** le trinitrate de glycéryle est absorbé sur le lactose à une concentration allant de 5 à 20% en poids.

5. Utilisation selon l'une quelconque des revendications précédentes, **caractérisée en ce que** la composition comprend aussi un stabilisant physique.

6. Utilisation selon la revendication 5, **caractérisée en ce que** la composition comprend aussi de l'eau.

7. Utilisation selon l'une quelconque des revendications précédentes, **caractérisée en ce que** la composition consiste essentiellement en 1% en poids de trinitrate de glycéryle (10% en poids de trinitrate de glycéryle absorbé sur 10% en poids de lactose), 44% en poids de lanoline, 21% en poids de paraffine blanche molle de la Pharmacopée Britannique et 25% en poids d'eau déminéralisée.

8. Utilisation selon l'une quelconque des revendications précédentes, **caractérisée en ce que** l'application topique est réalisée en conjonction avec l'utilisation d'un préservatif.

9. En combinaison avec un préservatif, composition pour application topique au pénis pour le traitement des troubles de la fonction érectile chez les mâles humains, **caractérisée en ce que** la composition comprend du trinitrate de glycéryle en tant qu'unique ingrédient actif et de la lanoline.
